# EUROPEAN PATENT APPLICATION

(11) **EP 4 321 518 A1**
(43) Date of publication of application: **14.02.2024**
(21) Application number: 22784426.3
(22) Date of filing: 14.03.2022
(51) Int. Cl.: C07H 13/08, A23L 33/105, A61K 8/60, A61K 8/9789, A61K 31/7024, A61K 36/48, A61P 17/00, A61P 43/00, A61Q 19/00

(54) **NOVEL POLYPHENOL COMPOUND**

(30) Priority: 09.04.2021 JP 2021066340
(71) Applicant: Nabocul Cosmetics Co., Ltd., Tokyo 101-0051 (JP)
(72) Inventor: TSIM, Karl Wah-Keung, Kowloon, Hong Kong (CN); YASHIRO, Takuya, Tokyo 101-0051 (JP); DONG, Tina Ting-Xia, Kowloon, Hong Kong (CN); PENG, Zhi-Tian, Shenzhen, (CN); WANG, Huai-You, Shenzhen, (CN); WEI, James, Tokyo 101-0051 (JP)
(74) Representative: Vossius & Partner Patentanwälte Rechtsanwälte mbB
(86) International application number: PCT/JP2022/011171
(87) International publication number: WO 2022/215441

(57) **Abstract**

Provided is a novel active ingredient derived from locust bean and the use thereof. The present invention is a compound represented by the following formula (I) or a salt thereof, or a solvate thereof.

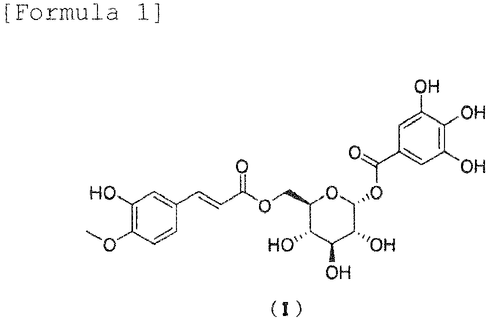

## Description

### Technical Field

The present invention relates to a novel polyphenol compound obtained by separation and purification from locust bean (*Ceratonia siliqua* L.) and the use of the compound as well as a method for producing the compound.

### Background Art

Locust bean (*Ceratonia siliqua L.*) is a leguminous plant mainly native to the Mediterranean region. The pod, that is, a shell and fruits, of locust bean is referred to as a carob and has been long used as food or a food ingredient. A mature pod has a length of about 10 to 25 cm and a sweet taste. The pod of locust bean contains a large amount of polysaccharides, cellulose and minerals, and a small amount of proteins and non-carbohydrate-based low-molecular compounds, and the like. In recent years, research on new functions of locust bean has been in progress, and it has been reported that an extract of pods of locust bean has preventive and therapeutic effects on gastrointestinal diseases such as ulcerative colitis and gastric ulcer (Non Patent Literatures 1 and 2). In addition, Patent Literature 1 discloses that a seed extract of locust bean has an α-glucosidase inhibitory activity and thereby has the effect of suppressing the body weight gain (Patent Literature 1).

In the meantime, collagen is an important protein that accounts for about 20% of the total proteins in the body. Collagen is mainly present in connective tissues and plays a role in giving strength, elasticity and extensibility to many tissues such as the cartilage, the bone, the tendon, the ligament, the dermis and the white part of the eye. Collagen is also a major constituent of the extracellular matrix. Dozens of collagen types are present depending on its structure. The dermis of the skin contains a large amount of type I collagen, which gives the skin strength and elasticity. The COL1A1 gene plays an important role in the expression and production of this type I collagen. The type I collagen is composed of two α1 chains encoded by the COL1A1 gene and one a2 chain encoded by the COL1A2 gene. Collagen plays a role in maintaining elasticity of the skin, and it is therefore known that a decrease in collagen with aging causes wrinkles in the skin. Accordingly, it has been believed that promoting the expression of collagen genes and promoting the in vivo production of collagen are effective means for compensating for the collagen that has decreased due to aging.

### Citation List

### Patent Literature

Patent Literature 1
Japanese Patent Laid-Open No. 2005-119999

### Non Patent Literature

Non Patent Literature 1
   Rtibi K, Jabri M A, Selmi S, et al., "Preventive effect of carob (Ceratonia siliqua L.) in dextran sulfate sodium-induced ulcerative colitis in rat", RSC Advances, 2016, Vol. 6, p.19992-20000.
Non Patent Literature 2
   Rtibi K, Selmi S, Grami. D, et al., "Chemical constituents and pharmacological actions of carob pods and leaves (Ceratonia siliqua L.) on the gastrointestinal tract: A review", Biomedicine & Pharmacotherapy, 2017, Vol. 93, p.522-528.

### Summary of Invention

### Technical Problem

However, Non Patent Literatures 1 and 2 and Patent Literature 1 report that the extract of locust bean has therapeutic effects on gastrointestinal diseases such as ulcers and the effect of suppressing the body weight gain. However, a specific active ingredient thereof has not yet been identified.

The use of locust bean for promoting the expression of collagen genes has not been previously studied, and thus its effectiveness has been completely unclear.

Accordingly, the present invention has been made in view of the above points. An object of the present invention is to provide a novel active ingredient derived from locust bean and the use thereof.

Another object of the present invention is to provide a novel collagen production-promoting agent, derived from locust bean, which is capable of promoting the expression of collagen genes. A further object of the present invention is to provide a skin external preparation, a cosmetic, and a food and drink product for promoting collagen production, comprising this collagen production-promoting agent.

### Solution to Problem

The present inventors have separated a novel polyphenol compound from an extract of pods of locust bean, and have found that this novel compound has the effect of increasing the expression level of collagen genes. Based on these findings, the present inventors have completed the present invention.

In order to solve the above problems, the present invention is a compound represented by the following formula (I) or a salt thereof, or a solvate thereof.

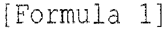

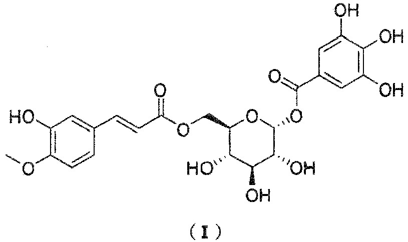

The compound represented by formula (I) is a novel polyphenol compound obtained by separation and purification from the extract of pods of locust bean (*Ceratonia siliqua*), and has an excellent collagen production-promoting effect.

The collagen production-promoting agent of the present invention comprises a compound represented by the above formula (I) or a salt thereof, or a solvate thereof. Administration of the compound represented by formula (I) can promote the expression of collagen genes and thereby promote the collagen production.

In the collagen production-promoting agent of the present invention, the concentration of the compound represented by the above formula (I) or a salt thereof, or a solvate thereof is also preferably 0.0001 mM to 1 mM. This enables the concentration of the active ingredient, having an excellent collagen production-promoting effect, to be selected.

The collagen production-promoting agent of the present invention is preferably a skin external preparation or a cosmetic. Thereby, it is possible to obtain a skin external preparation or a cosmetic that can promote the collagen production in the skin.

The food and drink product for promoting collagen production of the present invention comprises a compound represented by the above formula (I) or a salt thereof, or a solvate thereof. Thereby, it is possible to obtain a food and drink product that can promote the in vivo collagen production.

A method for producing a compound represented by the above formula (I) comprises: obtaining a hydroalcoholic extract of pods of locust bean (*Ceratonia siliqua*); subjecting the hydroalcoholic extract to liquid-liquid extraction with petroleum ether and ethyl acetate in this order to collect ethyl acetate fractions; and separating the compound represented by formula (I) from the collected ethyl acetate fractions. Thereby, it is possible to obtain a novel polyphenol compound having a collagen production-promoting effect.

### Advantageous Effects of Invention

The present invention can provide a novel polyphenol compound as well as a collagen production-promoting agent, a skin external preparation, a cosmetic and a food and drink product having the following excellent effects.
(1) The polyphenol compound can increase the expression level of collagen genes and thereby promote the collagen production.
(2) They are highly safe for the human body, because they have, as an active ingredient, a compound derived from pods of locust bean that have been edible since ancient times.

### Brief Description of Drawings

[Figure 1] Figure 1 is a diagram showing a high-resolution ESI mass spectrum of the compound of the present invention.
[Figure 2] Figure 2 is a diagram showing an ultraviolet absorption spectrum of the compound of the present invention.
[Figure 3] Figure 3 is a diagram showing an infrared absorption spectrum of the compound of the present invention.
[Figure 4] Figure 4 is a diagram showing a ¹H-NMR spectrum (CD₃OD, 600 MHz) of the compound of the present invention.
[Figure 5] Figure 5 is a diagram showing a ¹³C-NMR spectrum (CD₃OD, 150 MHz) of the compound of the present invention.
[Figure 6] Figure 6 is a diagram showing a HSQC spectrum of the compound of the present invention.
[Figure 7] Figure 7 is a diagram showing a HMBC spectrum of the compound of the present invention.

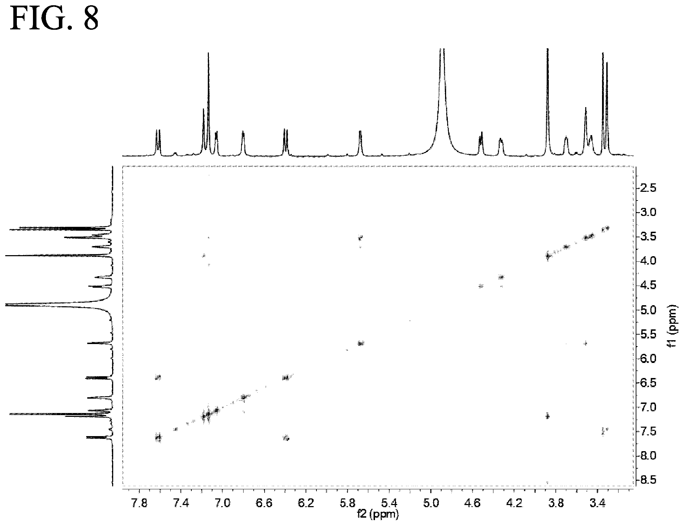

[Figure 8] Figure 8 is a diagram showing NOESY spectrum of the compound of the present invention.
[Figure 9] Figure 9 is a summary of ¹H-NMR signals and ¹³C-NMR signals of the compound of the present invention.
[Figure 10] Figure 10 is a diagram showing the HMBC correlation and the NOESY correlation in the compound of the present invention.
[Figure 11] Figure 11 is a graph showing the mRNA expression level of a collagen gene (COL1A1) by the compound of the present invention.

### Description of Embodiments

Hereinafter, a novel polyphenol compound of the present invention and a collagen production-promoting agent, and a skin external preparation, a cosmetic, and a food and drink product for promoting collagen production, comprising the polyphenol compound, as well as a method for producing the polyphenol compound will be described.

The novel polyphenol compound represented by the following formula (I) according to the present invention is a compound having each of isoferulic acid and gallic acid linked to glucose through an ester bond. This compound is named "OLANDU"(R).

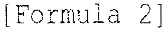

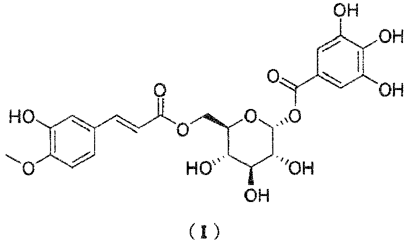

The novel polyphenol compound according to the present invention may be a salt thereof, and is preferably a pharmacologically acceptable salt. The pharmacologically acceptable salt of the novel polyphenol compound may be any salt formed together with an acid or base without limitation. The novel polyphenol compound or a salt thereof may be any solvate thereof. Examples thereof include, but are not particularly limited to, a hydrate and an organic solvate such as a solvate with ethanol.

The novel polyphenol compound according to the present invention "OLANDU" has a collagen production-promoting effect and can be used as a collagen production-promoting agent. The collagen whose production is promoted is preferably type I collagen or type III collagen which is rich in the skin, and more preferably type I collagen.

In the context of the present invention, the term "collagen production-promoting" or "promoting collagen production" refers to promoting the expression of a collagen gene or promoting the expression of collagen (protein), and it means that the expression of the collagen gene or collagen is enhanced compared to the control without the novel polyphenol compound of the present invention added or administered. More specifically, the expression level of the collagen gene is preferably 1.5 times or more that of the control, more preferably 1.7 times or more, and particularly preferably 2 times or more. The expression level of the collagen gene can be measured by any known method such as real-time PCR (QPCR) or microarray, and the expression level of collagen can be measured by any known method such as immunostaining and Western blotting.

The novel polyphenol compound of the present invention "OLANDU" can be obtained by separation and purification from pods of locust bean. The locust bean used in the present invention has a scientific name of *Ceratonia siliqua,* and is a plant belonging to the family Fabaceae, the subfamily Caesalpinioideae, the genus Ceratonia. It is a plant mainly native to the Mediterranean region, but in the present invention, the production area and cultivation environment are not particularly limited and locust bean from any production area and cultivation environment can be used.

A method for separating the novel polyphenol compound of the present invention "OLANDU" will be described. First, a hydroalcoholic extract is obtained from pods of locust bean. In the context of the present invention, "hydroalcoholic extract of pods of locust bean" refers to an extract obtained by subjecting pods of locust bean to an extraction treatment by adding a hydrous alcohol as an extraction solvent. The pod of locust bean refers to a shell and fruits of "fruits with a shell of locust bean", and either one of the shell or the fruits can be used as an extraction material, but both the shell and the fruits are more preferably used. The extraction treatment is subjected to the as-harvested, i.e., fresh or dried pods of locust bean. However, the extraction treatment can be subjected to the pods of locust bean that have been subjected to various pretreatment in order to improve extraction efficiency or facilitate handling. Examples of the pretreatment include, but are not particularly limited to, drying treatment, crushing treatment and grinding treatment. The pods of locust bean that have been subjected to such a pretreatment may be also subjected to the extraction treatment to obtain an extract.

The alcohol constituting the hydrous alcohol used as an extraction solvent is not particularly limited as long as it can extract the polyphenol compound of the present invention. Examples of such an alcohol include ethanol, methanol, propanol, isopropanol, butanol and isobutanol. Among these, hydrous ethanol is preferably selected as an extraction solvent from the viewpoint of safety to the human body, extraction efficiency, and the like. The concentration of the hydrous alcohol is preferably 50 to 99%, more preferably 60 to 97%, and particularly preferably 70 to 95%. The extraction solvent can further comprise other ingredients unless they interfere with the extraction of the compound of the present invention.

The method of extracting with a hydrous alcohol is a method comprising extracting pods of locust bean by adding the hydrous alcohol as an extraction solvent to the pods and immersing the pods in the hydrous alcohol. For example, when the pods of locust bean are dry crushed material with a water content of less than 10%, the extraction solvent is preferably used in the amount of 5 to 10 parts by weight per 1 part by weight of the plant body. The extraction method may be any method such as extraction at room temperature, extraction by heating, extraction by pressurizing and heating, or subcritical extraction, but extraction by heating under reflux is preferable from the viewpoint of extraction efficiency. In order to increase the extraction efficiency, the extraction operation is preferably performed multiple times, and more preferably performed multiple times with different alcohol concentrations in the extraction solvent. Examples of the extraction method include, but are not particularly limited to, an extraction method comprising performing extraction under reflux with 95% ethanol one to five times followed by extraction under reflux with 70% ethanol one to five times. The extraction time is variously set, depending on the extraction method, the type of extraction material, the type of extraction solvent or the extraction temperature or the like. However, for example, when the extraction under reflux is performed with 70 to 95% ethanol, the extraction time for one extraction is preferably set to about 1 to 3 hours, and particularly preferably about 1.5 hours. After the extraction treatment described above, the residue is removed by decantation, centrifugation or filtration, or the like to obtain a hydroalcoholic extract of pods of locust bean. The obtained extract also includes a concentrate or a solid obtained by subjecting the extract to a treatment such as distillation under reduced pressure.

The hydroalcoholic extract of pods of locust bean obtained as described above may contain saccharides and the like that are contained in large amounts in the pods of locust bean. Therefore, for the purpose of removing these unnecessary components, the hydroalcoholic extract may be subjected to separation operation with an ion exchange resin. Specifically, 1 to 10 parts by weight of water is added to 1 part by weight of a hydroalcoholic extract of pods of locust bean and disperse the extract in water. The dispersion is then passed through a column packed with an ion exchange resin such as a macroporous adsorption resin to adsorb the polyphenol compound of the present invention and flow out and remove unnecessary components such as saccharides. Thereafter, the adsorbed polyphenol compound of the present invention is eluted with 95% ethanol or the like to collect fractions containing the polyphenol compound of the present invention.

Next, a further separation operation to which the hydroalcoholic extract of pods of locust bean or the collected fractions separated by the ion exchange resin described above are subjected will be described. The hydroalcoholic extract or the collected fractions are dispersed in an aqueous solvent and are subjected to solvent extraction with petroleum ether and ethyl acetate in this order. The aqueous solvent is not particularly limited as long as it can disperse the polyphenol compound of the present invention, but a 50% hydrous methanol is suitably used. After performing liquid-liquid extraction with petroleum ether/an aqueous solvent multiple times, liquid-liquid extraction with ethyl acetate/an aqueous solvent is performed multiple times. The ethyl acetate fractions collected by this solvent extraction operation contain the novel polyphenol compound of the present invention. The number of liquid-liquid extractions in each solvent system is preferably about 2 to 10, particularly preferably about 5.

The methyl acetate fractions obtained as described above can be purified by a conventional method to isolate the novel polyphenol compound of the present invention "OLANDU". Examples of the purification method includes normal phase chromatography, reverse phase chromatography, thin layer chromatography, gel filtration chromatography and high-performance liquid chromatography. Purification may be performed by using one of these chromatographies or two or more in combination. Carriers, elution solvents and the like used in each chromatography can be appropriately selected depending on the chromatography.

The polyphenol compound of the present invention, which has been separated and purified from pods of locus bean as described above may not be isolated as a pure substance, and may be used as a mixture containing other components derived from a locust bean raw material.

The novel polyphenol compound of the present invention "OLANDU" can be used as collagen production-promoting agent that promotes the expression of collagen genes and promotes the in vivo production of collagen. The compound of the present invention increases collagen in the target cells by promoting the collagen production.

The collagen production-promoting agent comprising the novel polyphenol compound of the present invention "OLANDU" can be used as a skin external preparation for increasing the amount of collagen contained in skin cells, preventing and improving aging of the skin, and keeping the skin in a stable condition. The collagen production-promoting agent of the present invention can be also used as a cosmetic having the actions of increasing the amount of collagen contained in skin cells, preventing and improving wrinkles or sagging of the skin, and keeping the skin in a healthy condition.

The dosage of collagen production-promoting agent of the present invention varies depending on the target collagen production-promoting effect, preventive or therapeutic effect, administration method or age, or the like, and cannot be determined in general. However, when used as an external preparation, the usual daily parenteral dosage is preferably 0.02 µg to 30 ing, more preferably 0.2 µg to 3 mg, and even more preferably 2 µg to 300 µg of the polyphenol compound of the present invention. When used as an internal preparation, the usual daily oral dosage is preferably 0.2 µg to 1000 mg, and more preferably 2 µg to 200 mg of the polyphenol compound of the present invention.

The dosage form of the collagen production-promoting agent, the skin external preparation and the cosmetic of the present invention is not particularly limited, and examples thereof include liquids such as low-viscosity liquids or lotions; emulsions, gels, pastes, creams, foams, packs, ointments, powders, aerosols and patches; and tablets, granules, capsules and liquids for internal application. The collagen production-promoting agent according to the present invention can be applied for any of a cosmetic, a quasi-drug and a pharmaceutical. Specific examples of the cosmetic, quasi-drug and pharmaceutical include, but are not particularly limited to, cosmetic lotions, cosmetic creams, cosmetic emulsions, serums, cosmetic packs, cosmetic cleansers, soaps, hair care agents, bath agents, and makeup cosmetics.

The blending concentration of the novel polyphenol compound of the present invention contained in the collagen production-promoting agent, the skin external preparation and cosmetic is preferably 0.0001 mM to 1 mM, more preferably 1 µM to 100 µM, and even more preferably 5 µM to 20 µM. When the blended amount of the novel polyphenol compound of the present invention is within this range, the compound can be stably blended, it can be highly safe for the skin and it can exert a high collagen production-promoting effect.

The collagen production-promoting agent of the present invention can be prepared in various forms by any method conventionally used. In this case, it can be formulated with additives that are usually accepted as pharmaceutical additives, such as carriers and excipients for formulations. In addition, in order to improve the bioavailability and stability of the present compound, a drug delivery system including a formulation technique such as microencapsulation, pulverization or inclusion with cyclodextrin or the like can be also used.

The collagen production-promoting agent, the skin external preparation and the cosmetic of the present invention can comprise appropriately an ingredients commonly used in an external skin preparations and cosmetics, such as water, fats and oils, waxes, hydrocarbons, fatty acids, higher alcohols, esters, plant extracts, vitamins, water-soluble polymers, surfactants, metallic soaps, alcohols, polyhydric alcohols, pH adjustors, preservatives, fragrances, powders, thickeners, pigments and chelating agents. In addition, one or two or more of various functional ingredients usually used, for example, selected from moisturizing agents, whitening agents, anti-inflammatory agents, cell activators, ultraviolet protection agents, blood circulation promoters and antioxidants and the like can be used in combination with the compound of the present invention, unless they impair the actions and effects of the present invention.

The food and drink product for promoting collagen production of the present invention also comprises, as an active ingredient, the novel polyphenol compound of the present invention "OLANDU". The food and drink product for promoting collagen production of the present invention can be prepared in any form, for example, in the form of supplements such as tablets, capsules, granules and syrups; drinks such as soft drinks, fruit juice drinks, and alcoholic drinks; and sweets such as candies, gums, cookies, biscuits and chocolates; breads, porridges, cereals, noodles, jellies, soups, dairy products and seasonings. When used as food or drink products in this way, the food and drink product for promoting collagen production can be combined in various manner with various other active ingredients, or nutrients such as vitamins, minerals and amino acids unless they affect the efficacy of the active ingredient of the present invention. The food and drink product of the present invention include supplements, health foods, functional foods, foods for specified health uses, and the like. The daily intake of the food and drink product of the present invention is preferably 0.2 µg to 1000 mg and more preferably 2 µg to 200 mg of the polyphenol compound of the present invention.

Hereinafter, the present invention will be described in detail with reference to examples thereof, but the present invention will not be limited by these examples.

### Examples

### [Example 1]

### 1. Preparation of hydroalcoholic extract of pods of locust bean.

Seeds were removed from fruits with shells of locust bean (*Ceratonia siliqua*) that had been subjected to drying treatment after harvesting. Pods of this locust bean was ground with a grinder to obtain a ground product having a particle size of 2 mm or less. The operation of adding, to 20 kg of the ground product, 140 kg (7 times the amount of the ground product) of a 95% hydrous ethanol and extracting under refluxing for 1.5 hours was performed twice. Thereafter, the residue was further extracted under reflux with 140 kg (7 times the amount of the ground product) of a 70% hydrous ethanol for 1.5 hours. The extracts obtained by extraction under reflux were combined, and the solvent was then distilled off under reduced pressure to obtain 12.4 kg of a hydrous ethanol extract of pods of locust bean.

### [Example 2]

### 2. Separation and purification of hydroalcoholic extract of pods of locust bean

The hydroalcoholic extract of pods of locust bean obtained in Example 1 was dispersed in 1 to 10 times the amount of water and adsorbed on an ion exchange resin (a macroporous adsorption resin D101, available from Cangzhou Bon Adsorber Technology Co., Ltd.). Impurities such as saccharides were removed by eluting them with 3 times the column volume of distilled water, elution was then performed with 3 times the column volume of a 95% hydrous ethanol, and the solvent was distilled off under reduced pressure to obtain 462.7 g of ethanol elution fractions (non-carbohydrate-based low molecular compound fractions). Next, the obtained ethanol elution fractions were dispersed in 1.0 L of 50% hydrous methanol, and the dispersion was subjected to liquid-liquid extraction with petroleum ether and ethyl acetate in this order each five times. Each solvent was distilled off under reduced pressure to obtain 28.4 g of petroleum ether fractions, 139.4 g of ethyl acetate fractions and 290.2 g of aqueous fractions, respectively.

Then, a 135.0 g portion of the ethyl acetate fractions was subjected to normal phase silica gel column chromatography (a column packing material: 200 to 300 mesh; a product available from Qingdao Haiyang Chemical Co., Ltd.), and elution and fractionation were performed with two developing solvents, that is, petroleum ether (P)/ethyl acetate (E) and dichloromethane (C)/methanol (M). As a result, 108 elution fractions were obtained. The obtained 108 elution fractions were subjected to identification with thin layer chromatography, and similar fractions were combined to obtain ten elution fractions A to J.

Next, the elution fraction F (8.5 g) and the elution fraction G (9.6 g) were subjected to reverse phase CDS column chromatography (a column packing material: 40 to 63 µm; a product available from Merck & Co.), and fractionation was performed by gradient elution with methanol: water = 15: 85 → 100: 0. The obtained elution fractions were subjected to identification with thin layer chromatography, and similar fractions were combined to obtain six elution fractions F1 to F6.

Next, the elution fraction F4 (4.2 g) was subjected to gel filtration chromatography (a column packing material: Sephadex LH-20), and elution was performed with dichloromethane: methanol = 1:1 to obtain five elution fractions F4a to F4e.

Then, the elution fraction F4c (1.1 g) was subjected to semi-preparative HPLC (Column I: YMC-Pack ODS-A, 250 × 20 mm, 5 µm) and separation was performed with methanol:water = 40:60 at a detection wavelength of 217 nm to obtain three fractions F4c1, F4c2 and F4c3. Among these fractions, the fraction F4c2 was subjected to semi-preparative HPLC (Column II: YMC-Pack ODS-A, 250 × 10 mm, 5 µm) and separation was performed with methanol:water = 36:64 at a detection wavelength of 217 nm to obtain 23.8 mg of a compound of the present invention (hereinafter referred to as "OLANDU") (retention time tR = 37.14 minutes).

### [Example 3]

### 3. Structural analysis of compound (OLANDU)

The compound obtained in Example 2, "OLANDU" was subjected to structural analysis. For structural analysis, high-resolution mass spectrometry (HR-ESI-MS; negative ion mode), ultraviolet absorption spectrometry, infrared absorption spectrometry and ¹H-NMR, ¹³C-NMR, HMBC, HSQC and NOESY analyses were performed. The following devices were used for these analyses.
- High-resolution mass spectrometry: an electrospray ionization quadrupole time-of-flight mass spectrometry device (a product available from Bruker Daltonics)
- Ultraviolet absorption spectrometry: an ultraviolet-visible spectrophotometer (UV-2401PC, a product available from Shimadzu Corporation)
- Infrared absorption spectrometry: FT-IR (NEXUS470, a product available from Thermo nicolet)
- NMR analysis: a 600 MHz nuclear magnetic resonance device (AVANCE III 600, a product available from Bruker)

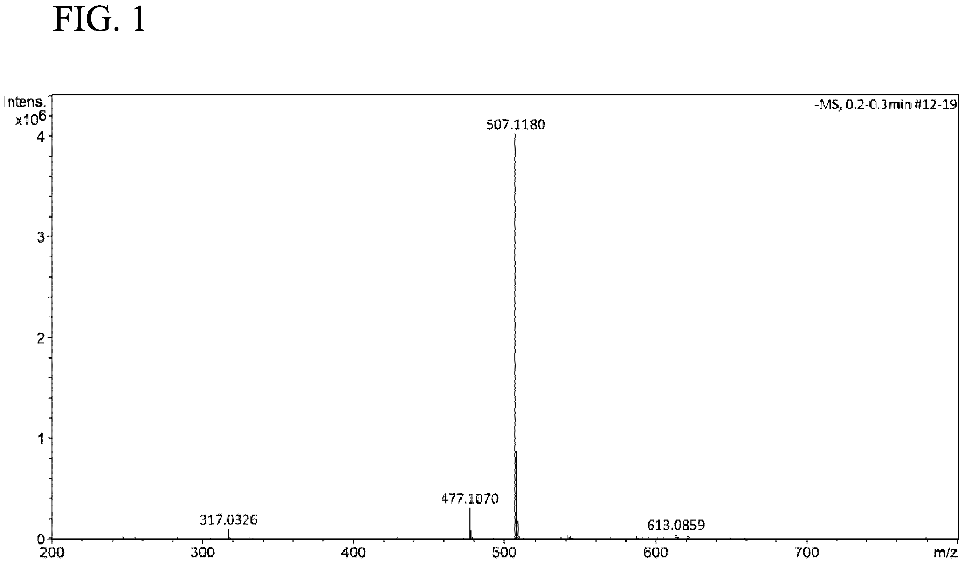

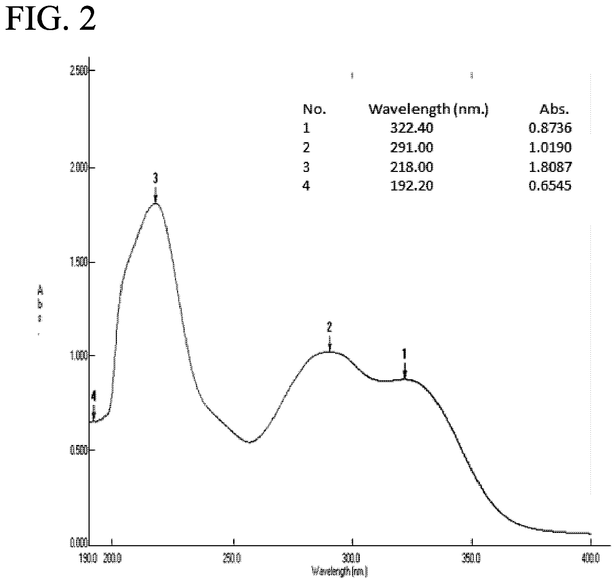

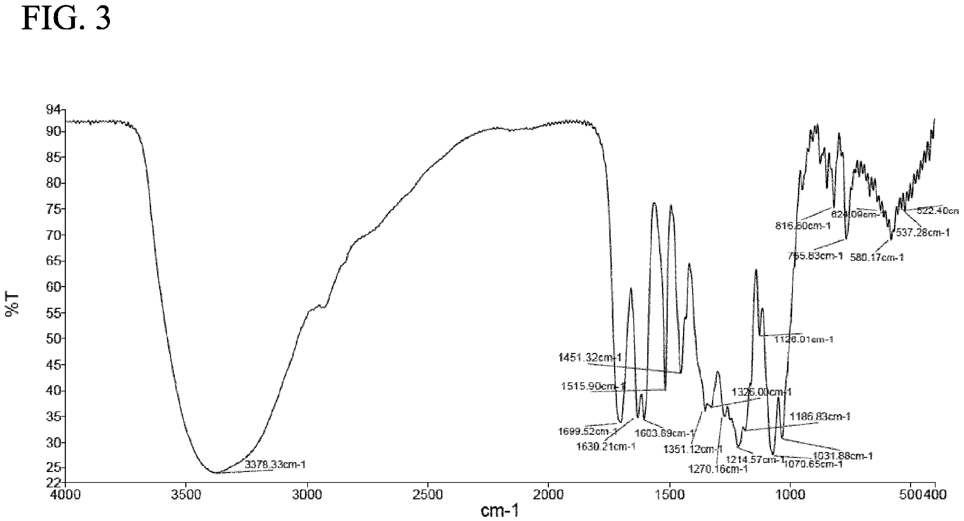

The physical properties of OLANDU are as follows. The spectrum of high-resolution mass spectrometry (HR-ESI-MS) is shown in Figure 1, the results of ultraviolet absorption spectrometry are shown in Figure 2, and the results of infrared absorption spectrometry are shown in Figure 3.
- Aspect: yellow powder
- HR-ESI-MS (negative) m/z: 507.1180 [M-H]⁻
- Ultraviolet absorption spectrum: λmax (MeOH) nm (log ε): 192.2 (3.82), 218.0 (4.26), 291.0 (4.02), 322.4 (3.95)
- Infrared absorption spectrum (KBr, cm⁻¹): vmax: 3378.33, 1699.52, 1630.21, 1603.89, 1515.90, 1451.32, 1351.12, 1326.00, 1270.16, 1214.57, 1186.83, 1126.01, 1070.65, 1031.88, 816.60, 765.83, 624.09, 580.17, 537.28, 522.40

The results of high-resolution mass spectrometry (Figure 1) showed that the quasi-molecular ion peak was m/z 507.1180 [M-H]⁻. Therefore, the compositional formula was estimated to be C₂₃H₂₄O₁₃, and the degree of unsaturation was estimated to be 12. From the infrared absorption spectrum (Figure 3), the molecule was presumed to include a hydroxyl group (3378.33 cm⁻¹), a benzene ring (1603.89 cm⁻¹, 1515.90 cm⁻¹) and an ester bond (1699.52 cm⁻¹, 1214.57 cm⁻¹).

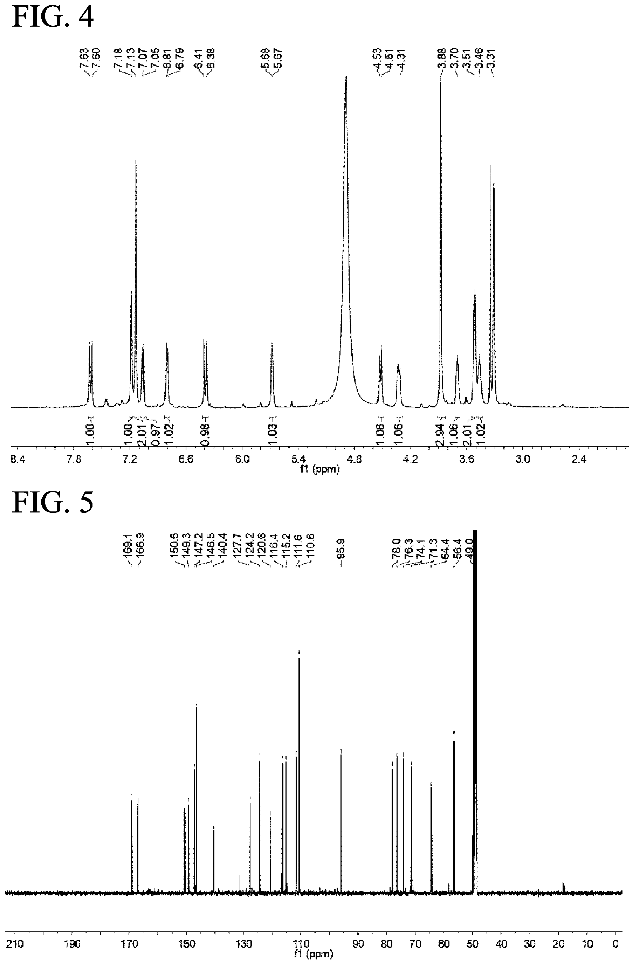

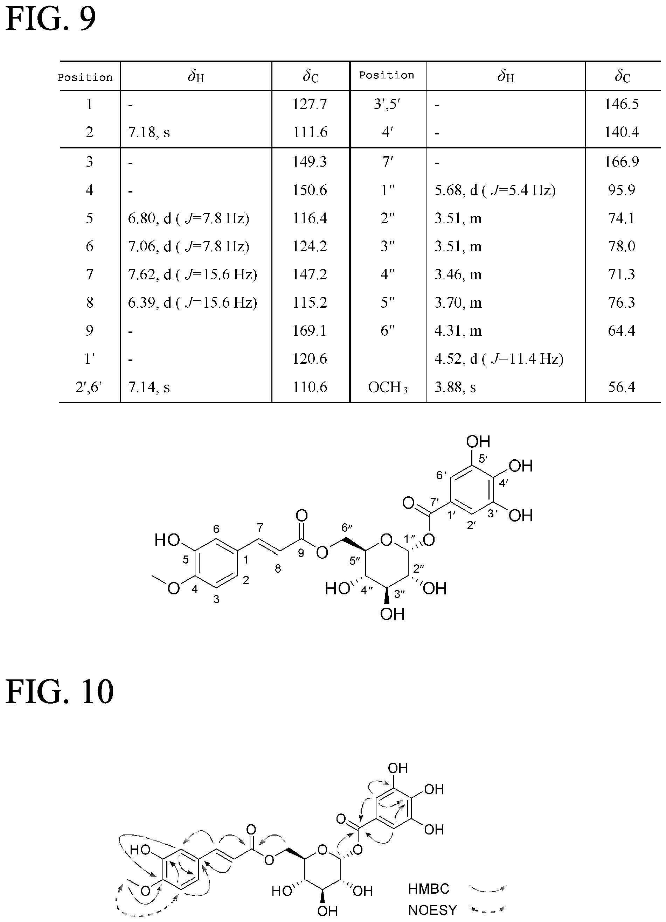

From the spectrum (Figure 4) obtained by ¹H-NMR analysis (a solvent: CB₃OD, observation frequency: 600 MHz), the following was confirmed (Figure 9): a set of trans-olefin proton signals [δ_{H}: 7.62 (1H, d, J = 15.6 Hz, H-7), 6.39 (1H, d, J = 15.6 Hz, H-8)]; a set of benzene ring ABX-based signals [δ_{H}: 7.18 (1H, s, H-2), 7.06 (1H, d, J = 7.8 Hz, H-6), 6.80 (1H, d, J=7.8 Hz, H-5)]; 1,3,4,5-substituted benzene ring hydrogen signals [δ_{H}: 7.14 (2H, s, H-2', H-6')]; one methoxy signal [δ_{H}: 3.88 (3H, s, OCH₃-3)], a set of glucose residue hydrogen signals [δ_{H}: 5.68 (1H, d, J = 5.4 Hz, H-1"), 4.52 (1H, d, J = 11.4 Hz, H-6α"), 4.31 (1H, m, H-6β"), 3.70 (1H, m, H-5"), 3.51 (2H, m, H-2", H-3"), 3.46 (1H, m, H-4")]. From the spectrum (Figure 5) obtained by ¹³C-NMR analysis (a solvent: CD₃OD, observation frequency: 150 MHz), 23 carbon signals were confirmed (Figure 9). Specifically, they included the following: two ester carbonyl carbon signals (δ_{C}: 169.1, 166.9); 14 aromatic or olefinic carbon signals (δ_{C}: 150.6, 149.3, 147.2, 146.5 × 2, 140.4, 127.7, 124.2, 120.6, 116.4, 115.2, 111.6, 110.6 × 2); one methoxy carbon signal (δ_{C}: 56. 4); and a set of glucose residue carbon signals (δ_{C}: 95.9, 78.0, 76.3, 74.1, 71.3, 64.4). From these analysis results, it was presumed that the compound "OLANDU" would be composed of one glucose residue unit, one ferulic acid unit and one gallic acid unit.

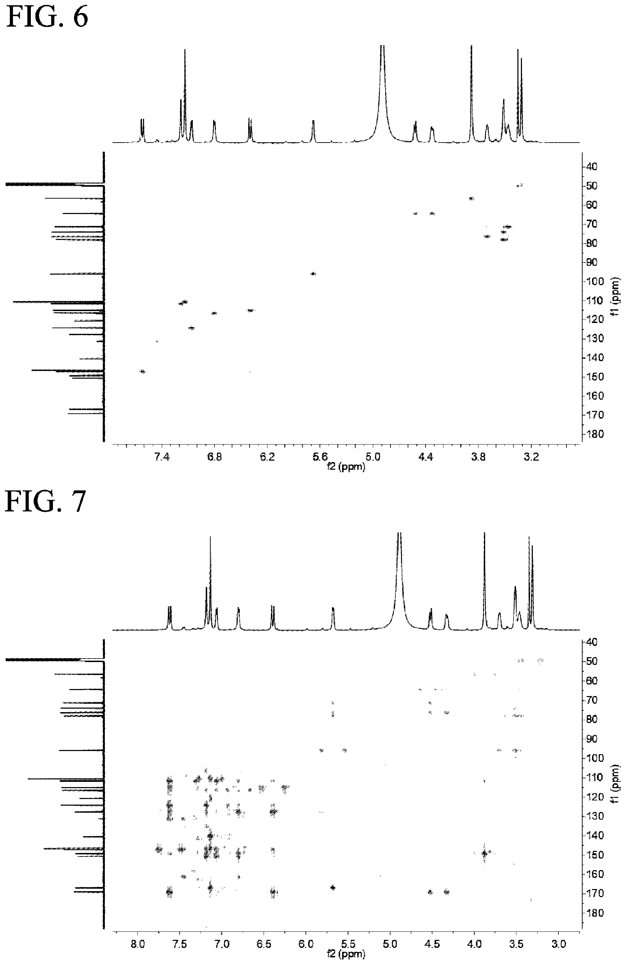

In addition, from the spectrum obtained by HMBC analysis (Figure 7), long-range correlation signals were confirmed between H-2 and C-4/C-6, between H-5 and C-1/C-3, between H-6 and C-4, between H-7 and C-2/C-6/C-9, between H-8 and C-1, and between OCH₃-3 and C-3. This result showed the presence of a ferulic acid unit in the structure of this compound. Further, long range correlation signals were confirmed among H-2', H-6' and C-4'/C-3'/C-7', indicating the presence of a gallic acid unit in the structure of this compound. Furthermore, long range correlation signals were confirmed between H-6" and C-9, indicating that ferulic acid was attached to C-6" of the glucose residue via an ester bond. In addition, long-range correlation signals between H-1" and C-7' indicated that gallic acid was attached to the terminal carbon of the glucose residue via an ester bond. The proton coupling constant of the glucose terminal was 5.4 Hz, indicating that the relative configuration of the glucose terminal was α-type. From these analysis results, the structure of this compound, "OLANDU" was determined to be represented by the following formula (I). Figure 9 shows an assignment table summarizing δ_{H} of ¹H-NMR signals and δ_{C} of ¹³C-NMR signals. Figure 10 shows the analysis results of NOESY and HMBC for OLANDU.

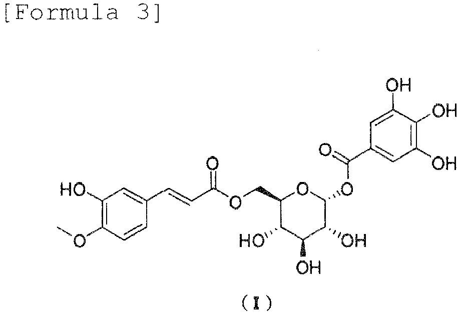

### [Example 4]

### 4. Study about action of promoting expression of collagen gene of OLANDU

The OLANDU obtained in Example 2 was used to examine the effect of promoting the expression of collagen gene (COL1A1 gene) in a HaCaT cell that is a human epidermis-derived keratinocyte cell line.

First, the HaCaT cells were cultured in a DMEM medium (containing 10% FBS, 100 U/mL penicillin and 100 µM/mL streptomycin) in the presence of 5% CO₂ at 37°C and saturated humidity. Next, the HaCaT cells adjusted to a cell concentration of 5 × 10⁵ cells/mL were seeded in each well of a 6-well cell culture plate and cultured in a CO₂ incubator (5% CO₂, 37°C) for 24 hours. Thereafter, the OLANDU isolated in Example 2 was added to each well to provide a final concentration different from each other. Specifically, the final concentrations of OLANDU in the HaCaT cell culture medium were 1 µM, 5 µM, 10 µM, 50 µM and 100 µM, respectively. A well to which the OLANDU had not been added was used as a control (0 µM). After addition, they were subjected to culture for 24 hours.

After completion of the culture, the HaCaT cells were collected from each well, and total RNA was extracted with an RNA extraction reagent (RNAzol(R), a product available from Molecular Research Center, Inc.). The concentration of total RNA obtained by extraction was confirmed with an ultratrace spectrophotometer (NanoDrop; a product available from Thermo Fisher Scientific). To this total RNA were added an oligo dT primer, a dNTP Mix, a 5 × 1st strand buffer, an RNase inhibitor, DTT and an M-MLV reverse transcriptase to synthesize cDNA from the total RNA. This cDNA was subjected to real-time PCR (QPCR) to measure the expression level of the collagen gene.

QPCR was performed with a commercially available QPCR reagent kit and QPCR measurement device (LightCycler(R) 480; a product available from Roche Diagnostics K. K.), and the detection was performed with SYBR(R) Green that is a double-stranded DNA-binding fluorescent dye. The COL1A1 gene encoding the α1 chain of type I collagen was selected as a target gene, and the primers of SEQ ID NOs: 1 and 2 shown in Table 1 below were used as QPCR primers.

**[Table 1]**

| | | |
|---|---|---|
| Forward primer | 5' - TCTGCGACAACGGCAAGGTG -3' | SEQ ID NO: 1 |
| Reverse primer | 5' - GACGCCGGTGGTTTCTTGGT -3' | SEQ ID NO: 2 |

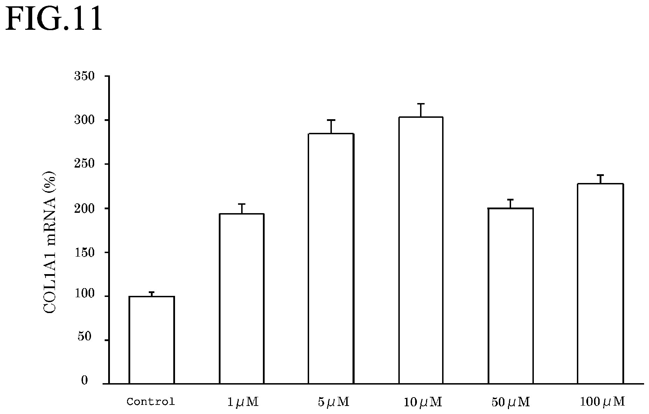

The results are shown in Figure 11. The mRNA expression level of the collagen gene (COL1A1 gene) is shown as a value relative to the expression level of a control of 100. Figure 11 clearly shows that the addition of the compound of the present invention OLANDU greatly increased the expression level of the collagen gene compared to the control without the OLANDU added. It was found that even when the OLANDU concentration was 1 µM, the expression level of the collagen gene increased approximately two-fold, and that at 10 µM, the expression level of the collagen gene increased more than three-fold, and the expression-promoting action was the highest.

The present invention is not limited to the above-described embodiments or examples, and includes various modifications without deviating from the scope of the invention described in the claims.

### Industrial Applicability

The novel polyphenol compound of the present invention is used as a collagen production-promoting agent, a skin external preparation and a cosmetic, and is widely used in the medical and cosmetic fields.

### Sequence Listing Free Text

SEQ ID NO: 1: target gene: a forward primer for QPCR of COL1A1
SEQ ID NO: 2: target gene: a reverse primer for QPCR of COL1A1

## Claims

1. A compound represented by the following formula (I) or a salt thereof, or a solvate thereof.

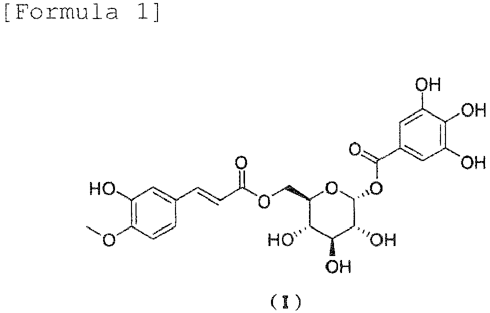

2. A collagen production-promoting agent comprising a compound represented by the following formula (I) or a salt thereof, or a solvate thereof.

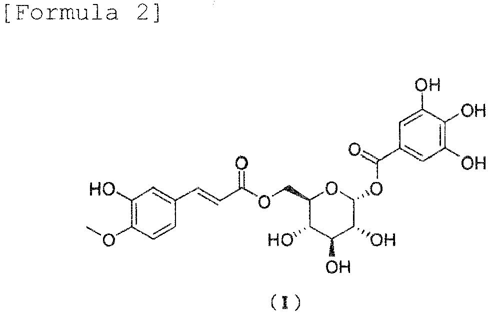

3. The collagen production-promoting agent according to claim 2, wherein the concentration of the compound represented by the following formula (I) or a salt thereof, or a solvate thereof is 0.0001 mM to 1 mM.

4. The agent according to claim 2 or 3, which is a skin external preparation.

5. The agent according to claim 2 or 3, which is a cosmetic.

6. A food and drink product for promoting collagen production comprising a compound represented by the following formula (I) or a salt thereof, or a solvate thereof.

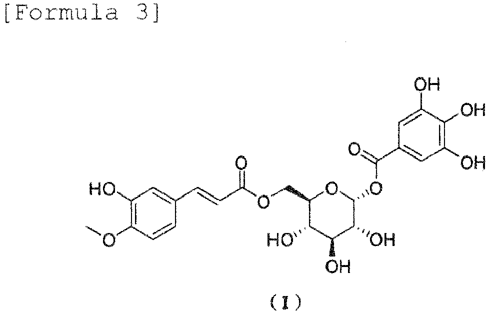

7. A method for producing a compound represented by the formula (I), comprising:
obtaining a hydroalcoholic extract of pods of locust bean (*Ceratonia siliqua*);
subjecting the hydroalcoholic extract to liquid-liquid extraction with petroleum ether and ethyl acetate in this order to collect ethyl acetate fractions; and
separating the compound from the ethyl acetate fractions.
